# EUROPEAN PATENT APPLICATION

(11) **EP 3 289 981 A1**
(43) Date of publication of application: **07.03.2018**
(21) Application number: 15891268.3
(22) Date of filing: 01.05.2015
(51) Int. Cl.: A61B 8/08

(54) **STOOL AMOUNT ESTIMATING APPARATUS AND STOOL AMOUNT ESTIMATING METHOD**

(71) Applicant: Triple W Japan K.K., Tokyo 1500031 (JP)
(72) Inventor: NAKANISHI, Atsushi, Tokyo 1500031 (JP); MASAMORI, Ryosuke, Tokyo 1500031 (JP)
(74) Representative: advotec.
(86) International application number: PCT/JP2015/002315
(87) International publication number: WO 2016/178261

(57) **Abstract**

A stool amount estimating apparatus 100 includes: an ultrasonic sensor 1 that detects a position of a wall of the urinary bladder 64; and an estimator 52 that estimates a stool amount accumulated in a rectum 67 based on an output of the ultrasonic sensor 1.

## Description

### TECHNICAL FIELD

The technique disclosed here relates to a stool amount estimating apparatus and a stool amount estimating method.

### BACKGROUND ART

Patent Document 1 describes a technique for predicting bowel movement of a subject by noninvasively detecting, from outside the body of the subject, that a detection target previously taken in the subject reaches the rectum.

### CITATION LIST

### PATENT DOCUMENT

PATENT DOCUMENT 1: Japanese Patent Application Publication No. 2009-247690

### SUMMARY OF THE INVENTION

### TECHNICAL PROBLEM

As described above, the technique described in Patent Document 1 detects bowel movement based on arrival of fecal matter at the rectum. The technique described in Patent Document 1, however, a subject needs to swallow a foreign substance such as a detection target. Thus, this technique involves pain and inconvenience. In cases where the subject is a person requiring care, he or she might not be able to swallow such a foreign substance smoothly.

In view of this, the inventor of the present invention focused on estimation of a stool amount. Estimation of the stool amount can raise the possibility of predicting bowel movement. In addition, the stool amount is information useful for various determinations and estimations as well as prediction of bowel movement.

It is therefore an object of the technique disclosed here to predict a stool amount accumulated in the rectum.

### SOLUTION TO PROBLEM

A stool amount estimating apparatus disclose here includes: a sensor that detects a position of a wall of a urinary bladder; and an estimator that estimates a stool amount accumulated in a rectum based on an output of the sensor.

A stool amount estimating method disclosed here includes the steps of: detecting a position of a wall of a urinary bladder; and estimating a stool amount accumulated in a rectum based on the detected position of the wall of the urinary bladder.

### ADVANTAGES OF THE INVENTION

The stool amount estimating apparatus can estimate a stool amount.

The stool amount estimating method can estimate a stool amount.

### BRIEF DESCRIPTION OF THE DRAWINGS

[FIG. 1] FIG. 1 is a block diagram of a stool amount estimating apparatus.
[FIG. 2] FIG. 2 is an illustration for describing the stool amount estimating apparatus in use.
[FIG. 3] FIG. 3 is a perspective view of the stool amount estimating apparatus 100.
[FIG. 4] FIG. 4 is a flowchart of bowel movement timing determination.
[FIG. 5] FIG. 5 is a schematic cross-sectional view mainly illustrating a lower abdomen of a human body.
[FIG. 6] FIG. 6 is an example of a received signal.
[FIG. 7] FIG. 7 is a schematic cross-sectional view mainly illustrating the lower abdomen of the human body in a state where the stool amount accumulated in the rectum is larger than that in the state of FIG. 5.
[FIG. 8] FIG. 8 is an example of a received signal in a state where the stool amount accumulated in the rectum is larger than that in the state of FIG. 6.
[FIG. 9] FIG. 9 is a flowchart of bowel movement timing determination according to a variation.
[FIG. 10] FIG. 10 is an example of a received signal subjected to signal processing for enabling detailed analysis of a reflected wave.
[FIG. 11] FIG. 11 is an example of a received signal subjected to signal processing similar to that in FIG. 10 in a case where an ultrasonic sensor 1 is attached at a level higher than that in FIG. 10.
[FIG. 12] FIG. 12 is a schematic cross-sectional view mainly illustrating a lower abdomen of a human body with a small urine volume in the urinary bladder.
[FIG. 13] FIG. 13 is an example of a received signal in a case where the urinary bladder cannot be detected by the ultrasonic sensor.

### DESCRIPTION OF EMBODIMENTS

An exemplary embodiment will be described in detail hereinafter with reference to the drawings.

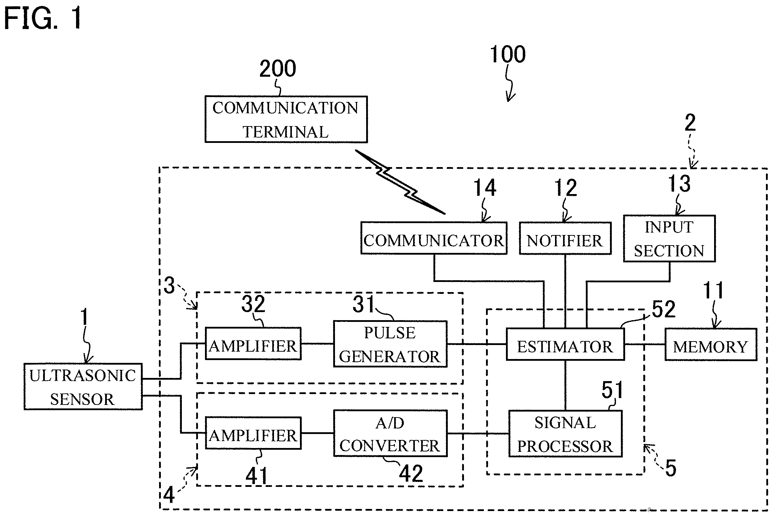

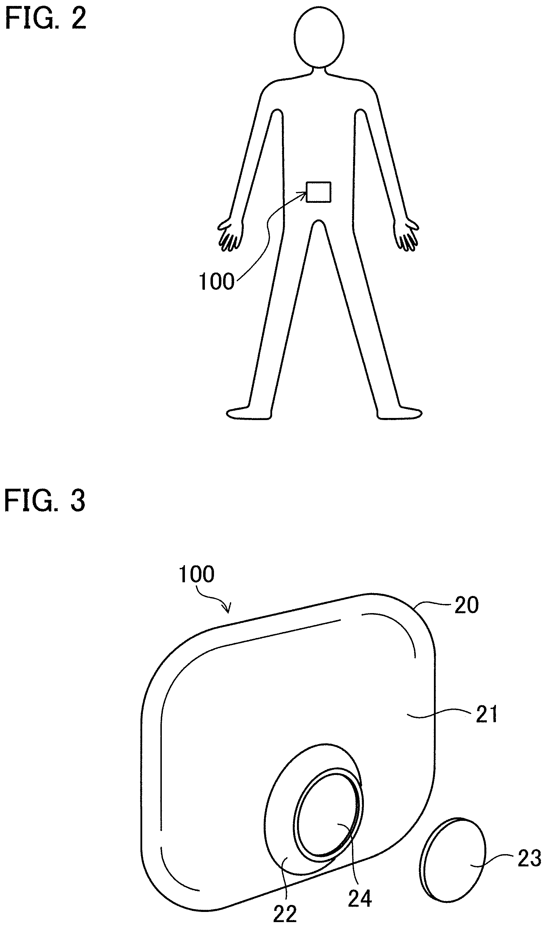

FIG. 1 is a block diagram of a stool amount estimating apparatus 100. FIG. 2 is an illustration of a state in which the stool amount estimating apparatus 100 is used. FIG. 3 is a perspective view of the stool amount estimating apparatus 100.

The stool amount estimating apparatus 100 estimates the stool amount accumulated in the rectum of a subject. Examples of the subject include those who require care, such as elderly people and physically disabled people, and those who do not require care but have difficulty in moving and need time for going to the lavatory. The subject, however, is not limited to these examples. The stool amount estimating apparatus 100 includes a casing 20, the ultrasonic sensor 1 for detecting the position of the wall of the urinary bladder, and an apparatus body 2 for controlling the ultrasonic sensor 1. The subject wears the stool amount estimating apparatus 100. Specifically, at least the ultrasonic sensor 1 is disposed on the skin of the abdomen of the subject in a portion corresponding to the urinary bladder (e.g., lower abdomen). The ultrasonic sensor 1 and the apparatus body 2 are housed in the casing 20 and are integrally configured.

The casing 20 has a substantially rectangular plate shape. A surface of the casing 20 in contact with the abdomen of the subject (hereinafter referred to as a "contact surface") 21 is provided with a projection 22 that incorporates the ultrasonic sensor 1. On the contact surface, the projection 22 is disposed substantially at the center in the lateral direction and a relatively lower portion in the vertical direction (below the center in the vertical direction). The front end of the projection 22 is provided with a gel pad 23 for increasing permeability of ultrasonic waves to the abdomen. More specifically, the front end of the projection 22 has a recess 24, and the gel pad 23 is disposed in the recess 24. The projection 22 contacts the abdomen of the subject with the gel pad 23 interposed therebetween.

The stool amount estimating apparatus 100 is worn by the subject by wrapping a belt over the abdomen of the subject so that the belt covers the stool amount estimating apparatus 100 with the contact surface 21 facing the abdomen of the subject and the projection 22 being in contact with the skin. Alternatively, the stool amount estimating apparatus 100 may be worn by the subject by attaching an adhesive tape to the abdomen of the subject so that the adhesive tape covers the stool amount estimating apparatus 100 with the contact surface 21 facing the abdomen of the subject and the projection 22 being in contact with the skin.

The ultrasonic sensor 1 transmits and receives ultrasonic waves. Specifically, the ultrasonic sensor 1 includes a transducer constituted by a piezoelectric element. The ultrasonic sensor 1 generates ultrasonic waves by generating vibrations in accordance with a driving voltage, and when receiving ultrasonic waves, generates an electrical signal in accordance with the vibrations thereof. The ultrasonic sensor 1 is an example of a sensor.

The apparatus body 2 includes a transmitter 3 that outputs the driving voltage to the ultrasonic sensor 1, a receiver 4 that receives the electrical signal from the ultrasonic sensor 1, a controller 5 that controls the entire stool amount estimating apparatus 100 and estimates a stool amount, a memory 11, a notifier 12 that issues various types of information to the outside, an input section 13 for inputting occurrence of actual bowel movement, and a communicator 14 that communicates with outside.

The transmitter 3 supplies the driving voltage to the ultrasonic sensor 1. The transmitter 3 includes a pulse generator 31 and an amplifier 32. The pulse generator 31 generates a pulse signal having a predetermined pulse width and a predetermined voltage value. The amplifier 32 amplifies the pulse signal from the pulse generator 31, and outputs the amplified pulse signal to the ultrasonic sensor 1 as the driving voltage.

The receiver 4 receives the electrical signal from the ultrasonic sensor 1. The receiver 4 includes an amplifier 41 and an analog-to-digital (A/D) converter 42. The amplifier 41 amplifies a signal received from the ultrasonic sensor 1 and outputs the amplified signal to the A/D converter 42. The A/D converter 42 performs A/D conversion on the signal received from amplifier 41 and outputs the resulting signal to the controller 5.

The communicator 14 communicates with an external communication terminal. In the stool amount estimating apparatus 100, an external communication terminal for communication can be registered, and the memory 11 stores information on this communication terminal. That is, a user registers a communication terminal 200 in the stool amount estimating apparatus 100 beforehand. In this manner, communication between the stool amount estimating apparatus 100 and the communication terminal 200 can be established. For example, a communication terminal 200 carried by a caregiver is registered in the stool amount estimating apparatus 100 beforehand. In cases where the subject can go to the lavatory by himself or herself, the communication terminal 200 of the subject is registered beforehand. The number of communication terminals 200 is not limited to one, and a plurality of communication terminals 200 (e.g., a communication terminal of a caregiver and a communication terminal of a person requiring care) may be registered.

The controller 5 includes one or more processors. The controller 5 controls the transmitter 3 and causes the transmitter 3 to output the driving voltage to the ultrasonic sensor 1, and estimates a stool amount based on a signal received from the receiver 4. The controller 5 includes a signal processor 51 that performs signal processing on the signal received from the receiver 4 and an estimator 52 that controls the transmitter 3 and estimates the stool amount.

The signal processor 51 performs signal processing such as averaging on the signal received from the receiver 4, and outputs the resulting signal to the estimator 52.

The estimator 52 issues an instruction for generating the pulse signal to the pulse generator 31 of the transmitter 3, and estimates the stool amount based on the received signal input from the signal processor 51. The transmitter 3 may be controlled by a section except the estimator 52.

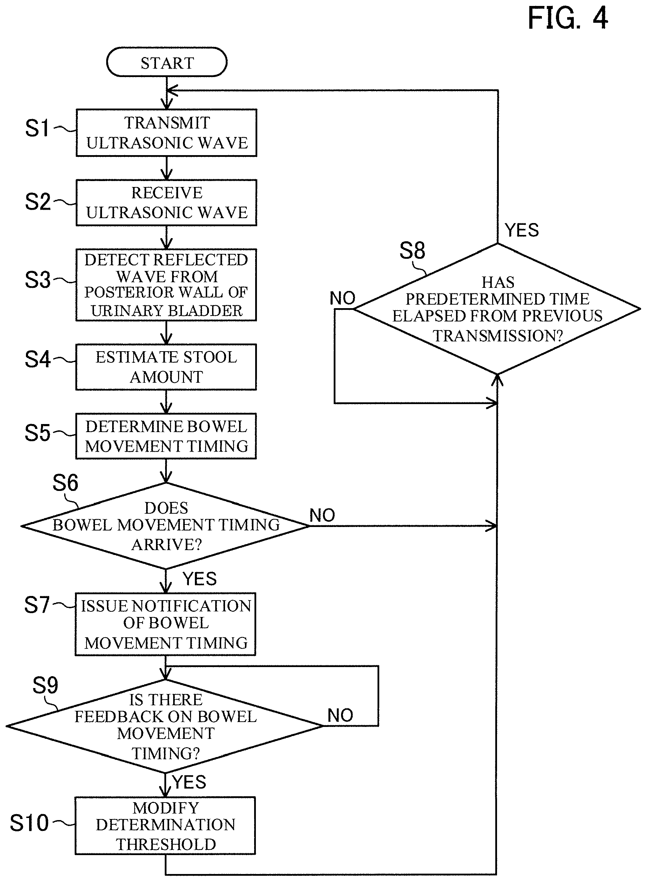

A process of the estimator 52 will now be described with reference to the flowchart of FIG. 4.

First, the estimator 52 outputs an instruction for transmitting ultrasonic waves (step S1). Specifically, the estimator 52 outputs an instruction for generating a pulse signal to the transmitter 3. This generation instruction serves as a trigger of an estimation process for a stool amount.

Thereafter, when the ultrasonic sensor 1 receives ultrasonic waves (i.e., when a received signal is input to the signal processor 51), the estimator 52 detects, based on the received signal, a reflected wave from the wall of the urinary bladder (step S3). Specifically, the estimator 52 detects the reflected wave from the posterior wall (wall toward the back) of the urinary bladder.

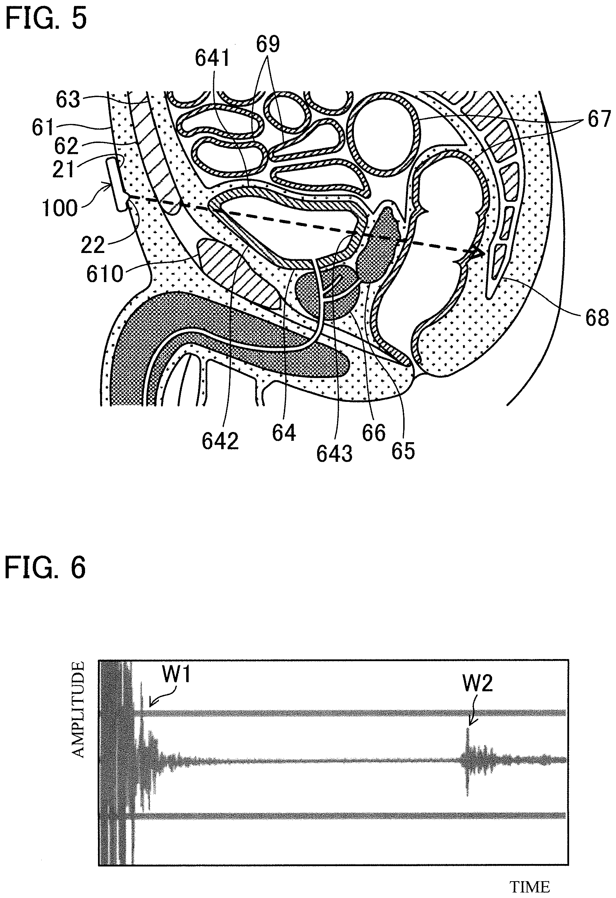

FIG. 5 is a schematic cross-sectional view mainly illustrating a lower abdomen of a human body. FIG. 6 is an example of the waveform of the received signal. The received signal shown in FIG. 6 is subjected to signal processing in the signal processor 51.

As illustrated in FIG. 5, subcutaneous fat 61, muscle 62, fat 63, urinary bladder 64, seminal vesicle 65 or prostate 66 (in a male) or vagina (in a female), rectum 67, backbone (sacrum) 68, and others are arranged in this order from the surface of the abdomen toward the back. Small intestine 69 is located above the urinary bladder 64, and pubis 610 is located obliquely below the front of the urinary bladder 64.

In the received signal in FIG. 6, a portion immediately after transmission is noise in transmission, and is a so-called dead zone. A reflected wave W1 immediately after the noise is a reflected wave from, for example, the subcutaneous fat 61, the muscle 62, and the fat 63 of the abdomen, and an upper wall 641 or an anterior wall (wall toward the abdomen) 642 of the urinary bladder 64. A reflected wave W2 is a reflected wave from a posterior wall 643 of the urinary bladder 64. The estimator 52 obtains a distance from the surface of the abdomen to the posterior wall 643 of the urinary bladder 64 based on a reception time of the reflected wave W2 (i.e., time from transmission of a transmission signal to reception of the reflected wave W2). The estimator 52 obtains the position of the posterior wall 643 of the urinary bladder 64 based on a reception time of a zero crossing point of the reflected wave W2 or a reception time of a peak (maximum value or minimum value) of the reflected wave W2.

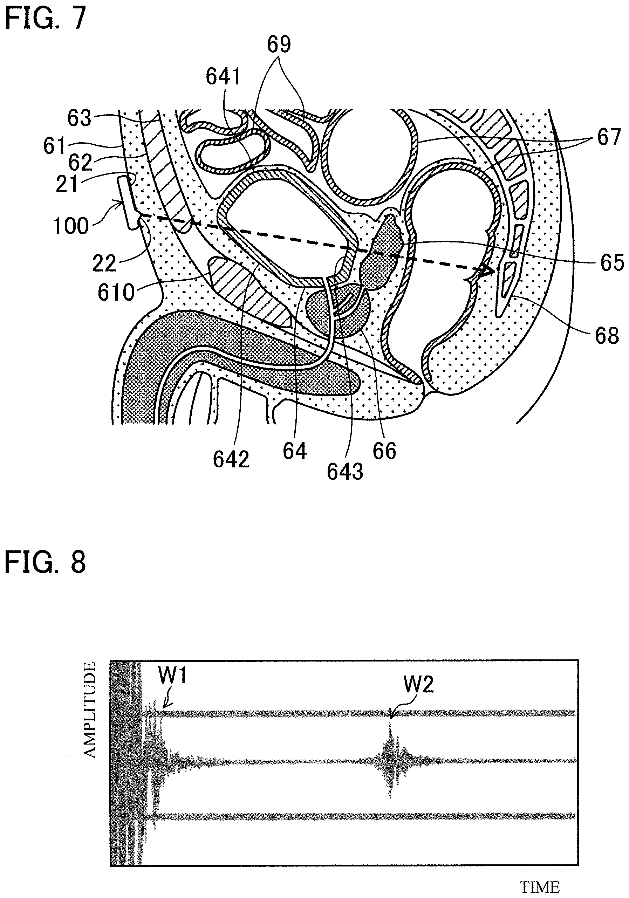

Subsequently, the estimator 52 estimates a stool amount based on the position of the posterior wall 643 of the urinary bladder 64 (step S4). FIG. 7 is a schematic cross-sectional view mainly illustrating the lower abdomen of the human body in a state where the stool amount accumulated in the rectum is larger than that in the state of FIG. 5. FIG. 8 shows an example of a received signal in a state where the stool amount accumulated in the rectum is larger than that in the state of FIG. 6. The received signal shown in FIG. 8 is subjected to signal processing in the signal processor 51.

As illustrated in FIG. 7, since the backbone 68 is present at the back of the rectum 67, when fecal matter is accumulated in the rectum 67, the rectum 67 expands forward to push the urinary bladder 64 forward under pressure. Consequently, the posterior wall 643 of the urinary bladder 64 moves forward. Accordingly, in the received signal of FIG. 8, the reception time of the reflected wave W2 that is a reflected wave from the posterior wall 643 of the urinary bladder 64 is earlier than that in FIG. 6. That is, as the stool amount accumulated in the rectum increases, the posterior wall 643 of the urinary bladder 64 moves forward, and accordingly, the received time of the reflected wave W2 is advanced. The estimator 52 obtains the position of the posterior wall 643 of the urinary bladder 64 based of the reception time of the reflected wave W2, and can estimate the stool amount based on the position of the posterior wall 643 of the urinary bladder 64. A relationship between the position of the posterior wall 643 of the urinary bladder 64 and the stool amount is preferably obtained and stored in the memory 11. Specifically, as the position of the posterior wall 643 of the urinary bladder 64 moves forward, the stool amount increases. The estimator 52 estimates the stool amount by using the obtained position of the posterior wall 643 of the urinary bladder 64 with reference to the relationship between the position of the posterior wall 643 of the urinary bladder 64 and the stool amount stored in the memory 11.

Thereafter, the estimator 52 determines a bowel movement timing based on the stool amount (step S5). Specifically, the estimator 52 determines whether the stool amount is greater than or equal to a predetermined determination threshold or not. The determination threshold is a stool amount when the bowel movement timing arrives. The bowel movement timing may be set at an arbitrary time (e.g., a time immediately before bowel movement or a time 10 minutes before bowel movement). A determination threshold corresponding to this time is set in the memory 11 beforehand. That is, the estimator 52 determines whether the stool amount reaches an amount assumed to correspond to the bowel movement timing or not.

The estimator 52 reads the determination threshold from the memory 11, and compares the readout determination threshold with the estimated stool amount (step S6). If the stool amount is greater than or equal to the determination threshold (i.e., YES), the estimator 52 determines that the bowel movement timing arrives and issues a notification of arrival of the bowel movement timing (step S7). Specifically, the estimator 52 causes the notifier 12 to operate and issues a notification of arrival of the bowel movement timing to a registered communication terminal 200 through the communicator 14. The notifier 12 is, for example, a vibrator. Vibrations of the vibrator notify the subject of arrival of the bowel movement timing. In this manner, the subject is previously notified of a defecation desire that is to arise in near future, and thus, can prepare for going to the lavatory.

The communication terminal 200 may include a downloaded application dedicated to the stool amount estimating apparatus 100 so that communication with the stool amount estimating apparatus 100 and an operation of the stool amount estimating apparatus 100 can be performed. When the communication terminal 200 receives a signal indicating arrival of the bowel movement timing from the stool amount estimating apparatus 100, the communication terminal 200 notifies a user carrying the communication terminal 200 of arrival of the bowel movement timing by, for example, displaying a notification of arrival of the bowel movement timing on a display. In this manner, the user carrying the communication terminal 200 is notified of arrival of the bowel movement timing so that guidance to the lavatory for the subject can be promoted.

On the other hand, if the stool amount is less than the determination threshold (i.e., NO), the estimator 52 determines whether a predetermined time has elapsed or not from the previous transmission of ultrasonic waves (step S8). If the predetermined time has elapsed, the estimator 52 returns to step S1 and transmits ultrasonic waves. On the other hand, if the predetermined time has not elapsed, the estimator 52 waits for a lapse of the predetermined time, and after the lapse of the predetermined time, returns to step S1. In this manner, the estimator 52 transmits ultrasonic waves in predetermined time intervals, and regularly performs determination of a bowel movement timing.

Every time the estimator 52 regularly performs determination of a bowel movement timing (a series of processes from an instruction for generating a pulse signal to determination), the estimator 52 stores the stool amount in the memory 11. In other words, the memory 11 stores a chronological change of stool amounts.

After having issued a notification of arrival of a bowel movement timing, the estimator 52 waits for a report on occurrence of actual bowel movement (step S9). The apparatus body 2 includes the input section 13. When bowel movement actually finishes, the input section 13 is operated by the subject or a third party such as a caregiver. The input section 13 is, for example, a push button. The report on occurrence of actual bowel movement can also be input from the communication terminal 200 to the stool amount estimating apparatus 100. When actual bowel movement finishes, a person carrying the communication terminal 200 operates the communication terminal 200 to transmit the report on occurrence of actual bowel movement to the stool amount estimating apparatus 100.

When receiving a signal from the input section 13 or the report on occurrence of actual bowel movement from the communication terminal 200, the estimator 52 determines that actual bowel movement occurred, and modifies the determination threshold (step S10). Specifically, based on a time from when a notification of arrival of a bowel movement timing is issued to when an input of a report on occurrence of actual bowel movement is received from the input section 13 or the communication terminal 200, the estimator 52 determines whether the notification of arrival of the bowel movement timing is issued at an appropriate time or not. A time required from when the stool amount reaches an amount corresponding to the determination threshold to when bowel movement occurs substantially coincides with the time from issuance of the notification to the report on bowel movement. In view of this, as long as the time difference between the time from issuance of the notification to the report on bowel movement and the time to bowel movement assumed as a bowel movement timing is within a predetermined range, the estimator 52 does not change the determination threshold. In such a case, since the estimator 52 issues the notification of the bowel movement timing at an appropriate time, the estimator 52 does not modify the determination threshold. On the other hand, if the time from issuance of the notification to the report on bowel movement is shorter than the assumed time to bowel movement below the predetermined range, the estimator 52 reduces the determination threshold by a predetermined amount. If the time from issuance of the notification to the report on bowel movement is longer than the assumed time to bowel movement beyond the predetermined range, the estimator 52 increases the determination threshold by a predetermined amount. In such cases, since the expected time necessary to bowel movement is different from an actual time necessary to bowel movement, the estimator 52 modifies the determination threshold so that the time difference decreases. The estimator 52 overwrites the modified determination threshold on the memory 11. The estimator 52 uses the modified determination threshold for subsequent determinations of bowel movement timings.

The width of the rectum varies among individuals. Similarly, the stool amount in the rectum immediately before bowel movement also varies among individuals. On the occurrence of actual bowel movement, the determination threshold is modified based on feedback so that a bowel movement timing can be accurately determined with a determination threshold depending on a subject.

When having finished modification of the determination threshold, the estimator 52 proceeds to step S8. That is, although step S8 has been described as a process after determination of a bowel movement timing in a case where a bowel movement timing has not arrived yet, in a case where the bowel movement timing has arrived, the estimator 52 also performs the process of step S8 eventually. In this manner, irrespective of whether a bowel movement timing arrives or not, the estimator 52 transmits ultrasonic waves at predetermined time intervals, and regularly performs determination of a bowel movement timing.

As described above, the stool amount estimating apparatus 100 includes the ultrasonic sensor 1 that detects the position of the wall of the urinary bladder 64 and the estimator 52 that estimates a stool amount accumulated in the rectum 67 based on an output of the ultrasonic sensor 1.

In other words, the stool amount estimating method described above includes the steps of detecting the position of the wall of the urinary bladder 64 and estimating the stool amount accumulated in the rectum 67 based on the detected position of the wall of the urinary bladder 64.

In this configuration, the ultrasonic sensor 1 detects the wall of the urinary bladder 64, specifically the position of the posterior wall 643. The rectum 67 is located rearward of the posterior wall 643 of the urinary bladder 64, and the backbone 68 is located rearward of the rectum 67. When fecal matter is accumulated in the rectum 67, the width of the rectum 67 increases. Since the backbone 68 is located rearward of the rectum 67, when the width of the rectum 67 increases, the rectum 67 expands forward rather than rearward. Consequently, the rectum 67 pushes and moves the seminal vesicle 65, the prostate 66, and the posterior wall 643 of the urinary bladder 64 forward. That is, the position of the posterior wall 643 of the urinary bladder 64 is related to the width of the rectum 67, and consequently the stool amount accumulated in the rectum 67. In view of this, the estimator 52 estimates the stool amount from the position of the posterior wall 643 of the urinary bladder 64 based on the output of the ultrasonic sensor 1. In this manner, the stool amount estimating apparatus 100 can accurately estimate the stool amount.

The estimator 52 estimates a bowel movement timing based on the estimated stool amount. For example, if the stool amount exceeds a predetermined determination threshold, the estimator 52 can determine that bowel movement will occur soon.

The stool amount estimating apparatus 100 further includes the notifier 12 that issues a notification of arrival of a bowel movement timing. The estimator 52 causes the notifier 12 to operate when the estimator 52 determines that a bowel movement timing arrives.

With this configuration, when the estimator 52 determines that a bowel movement timing arrives, the notifier 12 issues a notification of this arrival to an external device. In this manner, the subject wearing the ultrasonic sensor 1 or another third party can be notified of a bowel movement timing. For example, in cases where the subject is a person who has difficulty in moving and needs time for going to the lavatory, the subject can be previously notified of bowel movement before he or she feels a defecation desire by setting the time of issuing the notification of a bowel movement timing earlier. In this manner, the subject is encouraged to prepare for going to the lavatory relatively early. In cases where the subject is a person requiring care, the notification of bowel movement can be previously issued even before the caregiver feels a defecation desire. In this manner, a caregiver can guide the person requiring care to the lavatory with a sufficient margin of time.

In addition, the estimator 52 is configured to determine that a bowel movement timing arrives if the stool amount of the rectum is greater than or equal to a predetermined determination threshold, accept feedback on actual bowel movement after the determination of the bowel movement timing, and modify the determination threshold.

With this configuration, the estimator 52 determines a bowel movement timing based on the stool amount. However, since the width of the rectum varies among individuals, a determination threshold of the stool amount for use in determination of arrival of a bowel movement timing also varies among individuals. The estimator 52 accepts feedback on actual bowel movement after the determination of determination timing, and modifies the determination threshold. In this manner, the determination threshold is repeatedly modified depending on the subject. Consequently, a determination accuracy in determining a bowel movement timing by the estimator 52 can be enhanced.

Specifically, the estimator 52 is configured to accept an input indicating occurrence of actual bowel movement, and modifies the determination threshold based on a time from when the notification of a bowel movement timing is issued to when the input indicating occurrence of actual bowel movement is accepted.

In this manner, the determination threshold can be modified based on actual bowel movement.

In addition, since the ultrasonic sensor 1 is disposed on the projection 22 described above, adhesion to the skin (body surface) of the subject increases to promote entry of ultrasonic waves into the human body. In this manner, the detection capacity of the urinary bladder described later can be enhanced.

In addition, since the projection 22 is disposed on a relatively lower portion the contact surface 21, strangeness of the subject in waring the stool amount estimating apparatus 100 can be reduced. That is, to detect the posterior wall 643 of the urinary bladder 64, ultrasonic waves are preferably emitted from a relatively lower portion on the lower abdomen. If the wearing position of the stool amount estimating apparatus 100 is excessively low on the lower abdomen, the subject feels strangeness. On the other hand, since the projection 22 is located on a relatively lower portion of the contact surface 21 in the configuration described above, even when the projection 22 is disposed on a lower portion of the lower abdomen, the stool amount estimating apparatus 100 can be disposed on an upper portion as much as possible as a whole. Since the ultrasonic sensor 1 is incorporated in the projection 22, ultrasonic waves can be emitted from a relatively lower portion of the lower abdomen with reduced strangeness of the subject.

### Other Embodiments

In the foregoing section, the embodiment has been described as an example of the technique disclosed in the present application. The technique disclosed here, however, is not limited to this embodiment, and is applicable to other embodiments obtained by changes, replacements, additions, and/or omissions as necessary. Components described in the above embodiment may be combined as a new exemplary embodiment. Components provided in the accompanying drawings and the detailed description can include components unnecessary for solving problems as well as components necessary for solving problems in order to exemplify the technique. Therefore, it should not be concluded that such unnecessary components are necessary only because these unnecessary components are included in the accompanying drawings or the detailed description.

The above embodiment may be configured as described below.

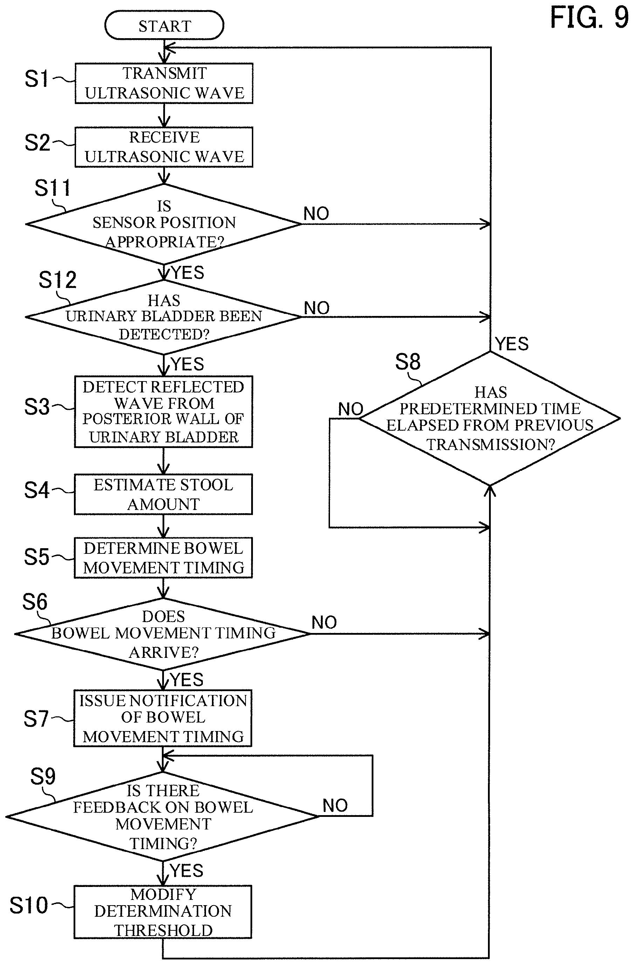

The stool amount estimating apparatus 100 transmits ultrasonic waves (step S1), and when receiving the ultrasonic waves (step S2), detects reflected waves from the posterior wall 643 of the urinary bladder 64 (step S3). At this time, before detecting the reflected waves from the posterior wall 643 of the urinary bladder 64, the stool amount estimating apparatus 100 may determine whether the subject wears the ultrasonic sensor 1 at an appropriate position or not and/or the urinary bladder 64 can be detected or not. FIG. 9 shows a flowchart in this case.

In the flowchart shown in FIG. 9, after receiving the ultrasonic waves in step S2, the estimator 52 determines whether the subject wears the ultrasonic sensor 1 at an appropriate position or not in step S 11.

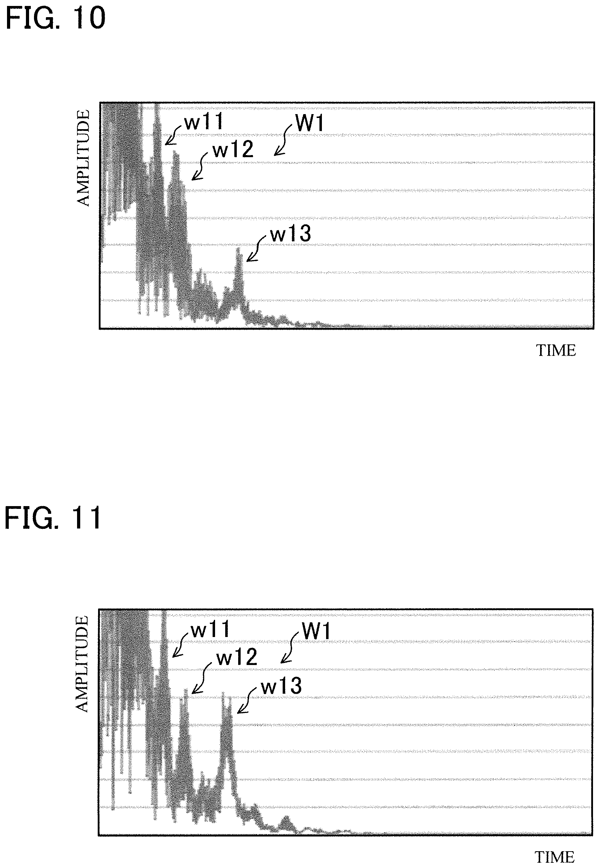

Specifically, based on the reflected wave W1 immediately after noise in a received signal, the estimator 52 determines whether the subject wears the ultrasonic sensor 1 at an appropriate position or not. FIG. 10 shows a received signal subjected to signal processing for enabling detailed analysis of the reflected wave W1. FIG. 11 shows a received signal subjected to signal processing similar to that in FIG. 10 in a state where the ultrasonic sensor 1 is disposed at a position higher than that in FIG. 10.

Since ultrasonic waves are reflected at the boundary between media having different acoustic impedances, the ultrasonic waves transmitted from the ultrasonic sensor 1 are also reflected on the surfaces of the subcutaneous fat 61, the muscle 62, and the fat 63. A more detailed analysis of the reflected wave W1 shows that the reflected wave W1 includes a reflected wave w11 from the subcutaneous fat 61, a reflected wave w12 from the muscle 62, and a reflected wave w13 from the fat 63. A comparison between FIGS. 10 and 11 shows that the time before each of the reflected waves w11 through w13 is received changes. As shown in FIG. 5, the thickness of each of the subcutaneous fat 61, the muscle 62, and the fat 63 varies depending on the vertical position. Based on the reception times of the reflected waves w11 through w13, the thicknesses of the subcutaneous fat 61, the muscle 62, and the fat 63 can be detected, and consequently, the wearing position of the ultrasonic sensor 1 can be estimated.

If the ultrasonic sensor 1 is disposed at an excessively high position, the posterior wall 643 of the urinary bladder 64 might fail to be appropriately detected. That is, the shape and size of the urinary bladder 64 change in accordance with the accumulated urine volume. If the urine volume is small, the urinary bladder 64 is small, and is located at a relatively low position. If the ultrasonic sensor 1 is disposed at an excessively high position, the posterior wall 643 might fail to be detected in a case where the urinary bladder 64 is small. On the other hand, if the ultrasonic sensor 1 is disposed at an excessively low position, the ultrasonic wave transmitted from the ultrasonic sensor 1 is reflected on the pubis 610 so that only a small component can reach the posterior wall 643 of the urinary bladder 64. Consequently, the amplitude of the reflected waves from the posterior wall 643 decreases, resulting in difficulty in detecting the reflected waves.

In view of this, based on the reflected waves w11 through w13, the estimator 52 estimates the thickness or proportion of each of the subcutaneous fat 61, the muscle 62, and fat 63. Based on this estimation, the estimator 52 determines whether the ultrasonic sensor 1 is disposed at an appropriate position or not. The thickness or proportion of each of the subcutaneous fat 61, the muscle 62, and fat 63 when the ultrasonic wave sensor 1 is disposed at the appropriate position is obtained beforehand, and is stored in the memory 11. The estimator 52 performs the determination by using the thicknesses or proportions of the subcutaneous fat 61, the muscle 62, and fat 63 estimated based on the reflected waves w11 through w13 with reference to those stored in the memory 11. Alternatively, the estimator 52 may determine that the ultrasonic sensor 1 is disposed at an excessively low position based on whether the reflected waves from the pubis 610 are present or not.

If the ultrasonic sensor 1 is disposed at the appropriate position, the estimator 52 proceeds to the next step, whereas if the ultrasonic sensor 1 is disposed at an inappropriate position, the estimator 52 notifies the subject of thisby using the notifier 12 to promote modification of the wearing position of the ultrasonic sensor 1.

Since the thicknesses and proportions of the subcutaneous fat 61, the muscle 62, and fat 63 significantly vary among individuals, even if the ultrasonic sensor 1 is determined to be disposed at an inappropriate position, the estimator 52 may simply notify the subject of this and proceed to the next step.

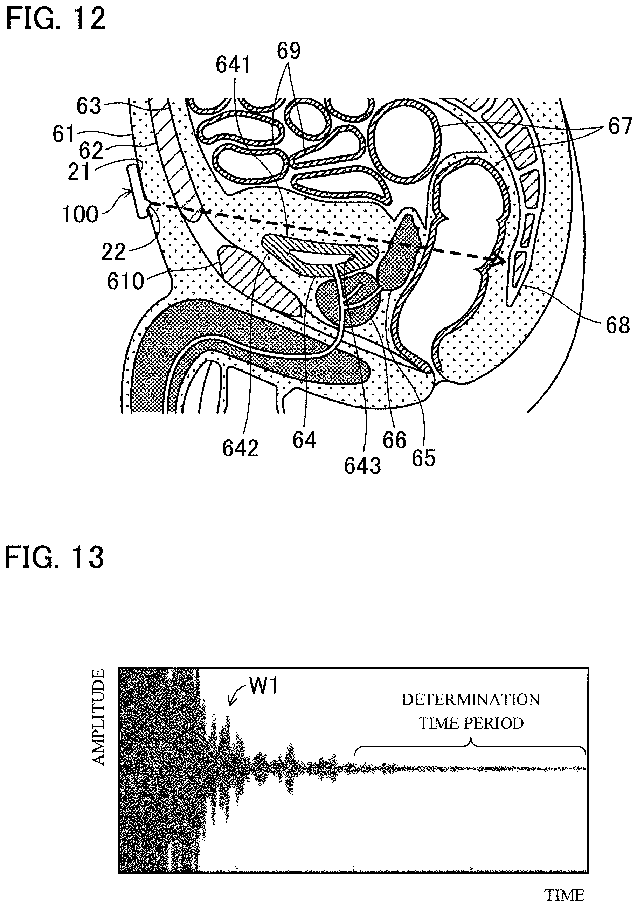

In addition, in step S12, the estimator 52 determines whether the urinary bladder 64 can be detected or not. As shown in FIG. 12, if the urine volume accumulated in the urinary bladder 64 is small, the urinary bladder 64 is small. If the urinary bladder 64 is small, the ultrasonic sensor 1 might fail to detect the wall of the urinary bladder 64, especially the posterior wall 643. In view of this, the estimator 52 detects whether the urinary bladder 64 is large enough to enable detection of the urinary bladder 64 by the ultrasonic sensor 1.

Specifically, the estimator 52 determines whether or not reflected waves are present in a reception time period (hereinafter referred to as a "determination time period") in which reflected waves from the posterior wall 643 of the urinary bladder 64 are supposed to return. FIG. 13 shows a received signal in a case where the urinary bladder 64 is too small to be detected by the ultrasonic sensor 1. In this received signal, no reflected waves can be observed in the determination time period. The estimator 52 determines whether the urinary bladder 64 can be detected or not based on whether the amplitude of reflected waves included in the determination time period is greater than or equal to a predetermined threshold or not.

The sensor for detecting the position of the posterior wall 643 of the urinary bladder 64 is not limited to the ultrasonic sensor 1. Any sensor except an ultrasonic sensor may be employed as long as the sensor can detect the posterior wall 643 of the urinary bladder 64. In the case of employing an ultrasonic sensor, the configuration of the ultrasonic sensor is not limited to the configuration described above. For example, an ultrasonic sensor including a plurality of transducers arranged in an array may be employed.

The method for wearing the stool amount estimating apparatus 100 is not limited to the method described above. For example, the contact surface 21 may be an adhesive surface so that the contact surface 21 is attached to the abdomen of the subject.

In the stool amount estimating apparatus 100, the ultrasonic sensor 1 and the apparatus body 2 are integrally provided. However, the configuration is not limited to this example. For example, the ultrasonic sensor 1 and the apparatus body 2 may be provided as separate components so that a subject wears only the ultrasonic sensor 1 and does not necessarily wear the apparatus body 2. In this case, the ultrasonic sensor 1 and the apparatus body 2 communicate with each other by wires or wirelessly. An apparatus worn by the subject includes at least the ultrasonic sensor 1 and may include other components. For example, the transmitter 3 and the receiver 4 may be separated from the apparatus body 2 to be included in an apparatus worn by the subject. In addition, the signal processor 51 may also be included in the apparatus worn by the subject. Furthermore, the controller 5 and the memory 11 may be included in the apparatus worn by the subject with only the notifier 12 and the input section 13 being separated from the apparatus worn by the subject.

An apparatus not worn by the subject may be constituted by a communication terminal such as a smartphone or a PC. For example, the ultrasonic sensor 1, the transmitter 3, the receiver 4, the controller 5, and the memory 11 may be integrally provided, and the notifier 12 and the input section 13 may be constituted by a communication terminal. In this case, the stool amount estimating apparatus 100 includes the ultrasonic sensor 1, the transmitter 3, the receiver 4, the controller 5, and the memory 11, and a communication terminal held by, for example, a caregiver functions as the notifier 12 and the input section 13. The stool amount estimating apparatus 100 determines a bowel movement timing as described above, and when the bowel movement timing arrives, the stool amount estimating apparatus 100 notifies the communication terminal of this arrival. In response to the notification of the bowel movement timing, the caregiver guides a person requiring care to the lavatory, and when actual bowel movement occurs, the caregiver transmits a report on this occurrence of actual bowel movement to the stool amount estimating apparatus 100 through the communication terminal. When the stool amount estimating apparatus 100 receives the report on the actual bowel movement, the stool amount estimating apparatus 100 modifies the determination threshold as described above.

The ultrasonic sensor 1, the apparatus body 2, and the communication terminal 200 such as a smartphone or a PC may form the stool amount estimating apparatus 100. In this case, functional parts of the transmitter 3, the receiver 4, the signal processor 51, and the estimator 52 not used for estimating the stool amount and the communicator 14 may be provided in the apparatus body 2, with the functional parts of the memory 11, the notifier 12, the input section 13, and the estimator 52 for estimating the stool amount being provided in the communication terminal 200. That is, the ultrasonic sensor 1 and the apparatus body 2 perform processes of transmitting ultrasonic waves, receiving reflected waves thereof, and performing signal processing on the received signal, and the received signal subjected to signal processing is transmitted from the apparatus body 2 to the communication terminal 200. The communication terminal 200 may be configured to receive the received signal and stores the received signal in the memory 11, perform operations such as estimation of a stool amount and prediction of a bowel movement timing by using the received signal, and performs notification when necessary.

The ultrasonic sensor 1, the apparatus body 2, the communication terminal 200 such as a smartphone or a PC, and a server may form the stool amount estimating apparatus 100. In this case, functional parts of the transmitter 3, the receiver 4, the signal processor 51, and the estimator 52 not used for estimating the stool amount and the communicator 14 may be provided in the apparatus body 2, with the functional parts of the memory 11 and the estimator 52 for estimating the stool amount being provided in the server, and the notifier 12 and the input section 13 being provided in the communication terminal 200. That is, the ultrasonic sensor 1 and the apparatus body 2 perform processes of transmitting ultrasonic waves, receiving reflected waves thereof, and performing signal processing on the received signal, and the received signal subjected to signal processing is transmitted from the apparatus body 2 to the server. The server receives the received signal and stores the received signal in the memory 11, and performs operations such as estimation of a stool amount and prediction of a bowel movement timing by using the received signal. The server transmits the stool amount and/or the bowel movement timing to the communication terminal 200, or if notification is necessary for the stool amount and/or the bowel movement timing, notifies the communication terminal 200 of this. The communication terminal 200 receives information on the stool amount and/or the bowel movement timing from the server, when necessary, displays the content of this information on a display device and causes an alarm or a vibrator to operate. As feedback of the bowel movement, notification of occurrence of the bowel movement is transmitted from the communication terminal 200 to the server by operation of the communication terminal 200.

A user may arbitrarily set a time interval defined as a margin immediately before bowel movement. For example, if the user inputs, as a bowel movement timing, a time that is five minutes before bowel movement, the estimator 52 sets, as a determination threshold, a stool amount assumed to be accumulated five minutes before the bowel movement. The memory 11 stores a relationship between a stool amount and the time to bowel movement with reference to an average width of the rectum. The estimator 52 sets, as a determination threshold, the stool amount depending on the input time.

The notifier 12 is not limited to a vibrator. The notifier 12 may be an alarm, a lamp, or a combination thereof. The notifier 12 may be a display device that displays an image or an animation in accordance with the content for notification. For example, the notifier 12 may display an image depending on the bowel movement timing on the display device. Specifically, the time before a bowel movement may be represented as an image of human facial expression that changes in such a manner that the degree of patience increases as the time before the bowel movement decreases.

Modification of the determination threshold may not be based on an input from the input section 13 and may be based on the determination value (i.e., the stool amount). For example, in a case where determination of a bowel movement timing is repetitively performed at short intervals, a change in stool amount in which the stool amount in the rectum increases, bowel movement occurs, and then the stool amount decreases is stored in the memory 11 in detail. In such a case, it can be determined when actual bowel movement occurred, based on the stool amounts stored in the memory 11. Based on this determination, the stool amount with which the time before bowel movement is a predetermined set time can be obtained. For example, actual bowel movement occurs at the time when the stool amount rapidly decreases from the maximum after issuance of a notification of arrival of a bowel movement timing. That is, the determination threshold may be modified based on the time from when the notification of the bowel movement timing is issued to when the stool amount reaches the maximum or the time until the stool amount rapidly decreases. In this manner, the determination threshold can be modified more accurately.

The repetitive intervals in regularly performing determination of a bowel movement timing may vary. For example, the determination may be performed in such a manner that determination of a bowel movement timing is performed in relatively long intervals (e.g., at every 10 minutes) before arrival of the bowel movement timing and estimation of the stool amount is performed in relatively short intervals (e.g., at every one minute) after determination of arrival of the bowel movement timing. In this case, the stool amounts before and after bowel movement can be more accurately detected and the determination threshold can be more accurately modified, with reduced power consumption.

In issuing a notification of arrival of a bowel movement timing, the estimator 52 may issue a notification of an approximate time before bowel movement. In this configuration, the determination threshold and a predicted time to bowel movement may also be modified based on actual bowel movement as described above.

The estimator 52 is not limited to a section that determines a bowel movement timing based on the value itself of the stool amount. For example, the estimator 52 may determine a bowel movement timing based on a change rate of the stool amount. It may be determined that fecal matter reaches the rectum, that is, a bowel movement timing arrives, based on a rapid increase in the stool amount. That is, the expression "based on the stool amount" in "estimating a bowel movement timing based on a stool amount" includes both "based on the value itself of the stool amount" and "based on a change rate of the stool amount."

In the above example, the transmitter 3 inputs a pulse signal to the ultrasonic sensor 1 as a driving signal. The driving signal, however, is not limited to a pulse signal. The driving signal may be a burst wave instead of a pulse wave.

A frequency modulated continuous wave may also be used as the driving signal. In this case, the estimator 52 analyzes the frequency of a received signal to detect the width of the rectum. A technique of the frequency analysis may be a fast Fourier transform (FFT) or a maximum entropy method (MEM).

The stool amount estimating apparatus 100 estimates a stool amount based on the position of the posterior wall 643 of the urinary bladder 64. The wall of the urinary bladder 64 to be detected, however, is not limited to the posterior wall 643. As described above, when the width of the rectum 67 increases with an increase of the stool amount, the posterior wall 643 of the urinary bladder 64 is pushed forward. At this time, not only the posterior wall 643 of the urinary bladder 64 but also the upper wall 641 of the urinary bladder 64 moves. That is, based on the position of the upper wall 641, the width of the rectum 67, and consequently the stool amount, can be estimated. Is should be noted that in consideration of the incident angle of ultrasonic waves from the ultrasonic sensor 1 to a human body, reflection angles from the walls of the urinary bladder 64, and the like, in the case of attaching the ultrasonic sensor 1 to the abdomen, detection can be more easily performed for the position corresponding to the posterior wall 643, than to the upper wall 641.

In addition, the estimator 52 estimates the stool amount based on the position of the wall of the urinary bladder 64 and predicts a bowel movement timing based on the stool amount. Alternatively, the estimator 52, may predict a bowel movement timing based on the position of the wall of the urinary bladder 64. That is, the position of the wall of the urinary bladder 64 is correlated with the stool amount, and the stool amount is correlated with the bowel movement timing. Accordingly, the position of the wall of the urinary bladder 64 is also correlated with the bowel movement timing. In view of this, relationships between the position of the wall of the urinary bladder 64 and a bowel movement timing may be previously obtained and stored in the memory 11. The estimator 52 may predict a bowel movement timing by detecting the position of the wall of the urinary bladder 64 and using the position of the wall with reference to the relationship between the position of the wall of the urinary bladder 64 and the bowel movement timing stored in the memory 11. In such a case, although a bowel movement timing is finally obtained, relationships among the position of the wall of the urinary bladder 64, the stool amount, and the bowel movement timing is used, and thus, it can be assumed that the stool amount is estimated substantially based on the position of the wall of the urinary bladder 64.

In the embodiment described above, the stool amount estimating apparatus 100 predicts a bowel movement timing based on the estimated stool amount, but does not need to predict the bowel movement timing. The stool amount estimating apparatus 100 may only estimate the stool amount, or may predict an event except for a bowel movement timing by using an estimated stool amount. For example, the timing of administrating a drug such as a laxative may be determined by determining whether fecal matter reaches the rectum or not based on a stool amount estimated by the stool amount estimating apparatus 100.

### DESCRIPTION OF REFERENCE CHARACTERS

- 100: stool amount estimating apparatus
- 1: ultrasonic sensor (sensor)
- 52: estimator
- 200: communication terminal

## Claims

1. A stool amount estimating apparatus comprising:
a sensor that detects a position of a wall of a urinary bladder; and
an estimator that estimates a stool amount accumulated in a rectum based on an output of the sensor.

2. The stool amount estimating apparatus of claim 1, wherein
the estimator determines a bowel movement timing based on the estimated stool amount.

3. A stool amount estimating method comprising the steps of:
detecting a position of a wall of a urinary bladder; and
estimating a stool amount accumulated in a rectum based on the detected position of the wall of the urinary bladder.

4. The stool amount estimating method of claim 3, further comprising the step of:
determining a bowel movement timing based on the estimated stool amount.
